(19) Europäisches Patentamt European Patent Office Office européen des brevets

(11) **EP 3 690 052 B1**

(12) **EUROPEAN PATENT SPECIFICATION**

(45) Date of publication and mention of the grant of the patent:
**19.04.2023 Bulletin 2023/16**

(21) Application number: **19154364.4**

(22) Date of filing: **29.01.2019**

(51) International Patent Classification (IPC):
**C12N 15/81** (2006.01)   **C12N 9/02** (2006.01)
**C12N 9/12** (2006.01)   **C12N 9/90** (2006.01)

(52) Cooperative Patent Classification (CPC):
**C12N 15/81; C12N 9/90;** Y02E 50/10

(54) **CHIMERIC PROMOTER FOR USE IN METABOLIC ENGINEERING**

CHIMÄRER PROMOTER ZUR VERWENDUNG IN DER METABOLISCHEN MANIPULATION

PROMOTEUR CHIMÉRIQUE POUR UNE UTILISATION DANS L'INGÉNIERIE MÉTABOLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**05.08.2020 Bulletin 2020/32**

(73) Proprietor: **Clariant Produkte (Deutschland) GmbH**
**65929 Frankfurt am Main (DE)**

(72) Inventors:
• **DIETZ, Heiko**
  **27639 Wurster Nordseeküste (DE)**
• **CLAREN, Jörg**
  **82131 Stockdorf (DE)**
• **FARWICK, Alexander**
  **81373 München (DE)**
• **MERTEL, Magdalena**
  **85247 Schwabhausen (DE)**

(74) Representative: **Graser, Konstanze**
**Clariant Produkte (Deutschland) GmbH**
**IPM / Patent & License Management**
**Arabellastrasse 4a**
**81925 München (DE)**

(56) References cited:
EP-A1- 0 511 912    WO-A1-2013/050551
WO-A1-2017/011184    WO-A2-2012/045088
US-A- 5 646 012

• ZAIN Y. DOSSANI ET AL: "A combinatorial approach to synthetic transcription factor-promoter combinations for yeast strain engineering", YEATS, vol. 35, no. 3, 1 March 2018 (2018-03-01), pages 273-280, XP055595628, ISSN: 0749-503X, DOI: 10.1002/yea.3292
• TOM ELLIS ET AL: "Diversity-based, model-guided construction of synthetic gene networks with predicted functions", NATURE BIOTECHNOLOGY, vol. 27, no. 5, 19 April 2009 (2009-04-19) , pages 465-471, XP055595805, New York ISSN: 1087-0156, DOI: 10.1038/nbt.1536
• ALBERT J.J. VAN OOYEN ET AL: "Heterologous protein production in the yeast Kluyveromyces lactis", FEMS YEAST RESEARCH, vol. 6, no. 3, 1 May 2006 (2006-05-01), pages 381-392, XP055529474, GB, NL ISSN: 1567-1356, DOI: 10.1111/j.1567-1364.2006.00049.x
• A. GARCÍA-LEIRO ET AL: "Proteomic Analysis of the Oxidative Stress Response in Kluyveromyces lactis and Effect of Glutathione Reductase Depletion", JOURNAL OF PROTEOME RESEARCH, vol. 9, no. 5, 7 May 2010 (2010-05-07), pages 2358-2376, XP055595543, ISSN: 1535-3893, DOI: 10.1021/pr901086w
• HUBMANN G ET AL: "Natural and Modified Promoters for Tailored Metabolic Engineering of the Yeast Saccharomyces cerevisiae", YEAST METABOLIC ENGINEERING: METHODS AND PROTO; [SERIES TITLE: METHODS IN MOLECULAR BIOLOGY , ISSN 1064-3745], HUMANA PRESS INC, US, vol. 1152, 1 January 2014 (2014-01-01), pages 17-42, XP009194186, DOI: 10.1007/978-1-4939-0563-8_2 ISBN: 978-1-4939-0562-1

EP 3 690 052 B1

**(Cont. next page)**

- **FABIAN MACHENS ET AL: "Synthetic Promoters and Transcription Factors for Heterologous Protein Expression in Saccharomyces cerevisiae", FRONTIERS IN BIOENGINEERING AND BIOTECHNOLOGY, vol. 5, 19 October 2017 (2017-10-19), XP055595708, DOI: 10.3389/fbioe.2017.00063**
- **VENKATESH ENDALUR GOPINARAYANAN ET AL: "A semi-synthetic regulon enables rapid growth of yeast on xylose", NATURE COMMUNICATIONS, vol. 9, no. 1, 26 March 2018 (2018-03-26), XP055596035, DOI: 10.1038/s41467-018-03645-7**
- **ZHANG XINYUAN ET AL: "Optimizing the coordinated transcription of central xylose-metabolism genes inSaccharomyces cerevisiae", APPLIED MICROBIOLOGY AND BIOTECHNOLOGY, SPRINGER, DE, vol. 102, no. 16, 26 June 2018 (2018-06-26), pages 7207-7217, XP036557450, ISSN: 0175-7598, DOI: 10.1007/S00253-018-9172-5 [retrieved on 2018-06-26]**
- **JEFFRIES ET AL: "Engineering yeasts for xylose metabolism", CURRENT OPINION IN BIOTECHNOLOGY, LONDON, GB, vol. 17, no. 3, 1 June 2006 (2006-06-01), pages 320-326, XP024962858, ISSN: 0958-1669, DOI: 10.1016/J.COPBIO.2006.05.008 [retrieved on 2006-06-01]**

Remarks:
The complete document including Reference Table(s) and the Sequence Listing(s) can be downloaded from the EPO website

**Description**

The present invention comprises a chimeric promoter which can initiate the transcription of a gene under various conditions such as varying carbon sources. Further the invention relates to a recombinant DNA fragment comprising the chimeric promoter, an expression plasmid comprising the recombinant DNA fragment and a host cell transformed with the recombinant DNA fragment.

The yeast *5. cerevisiae* and *S. sensu stricto* species are used since thousands of years for the production of bread and alcoholic beverages like sake, wine or beer. Through this long period of industrial usage, yeasts are adapted to the process conditions and can tolerate the mechanical forces in a bioreactor, inhibitory substances and fermentation products. Further they are robust against fluctuations in temperature and can ferment sugars at low pH-value, which minimizes the contamination risk. Besides this, *S. cerevisiae* is a key laboratory model system and can be easily genetically modified and is generally recognized as safe - GRAS status. A broad genetic tool set is available for *S. cerevisiae* and many intracellular processes like metabolism, secretion, transport, signaling and other pathways are well studied, which help to successfully engineer the yeast for a wide variety of applications.

Especially the introduction of multi-enzyme pathways requires the regulation and control over the gene expression depending on different conditions such as varying carbon sources including varying ratios of carbon sources, wherein the enzyme is heterologous or native, to optimize substrate utilization and/or product formation. Thereby the transcriptional control takes place at the oligonucleotide sequence which is located in the upstream region of a gene - the promoter. Thus, promoter strength and regulation are critical points for metabolic engineering.

An important factor in the metabolic engineering and the selection of promoters is genetic stability. Multiple use of the same promoters often results in genetic instability in engineered strains due to homologous recombination between stretches of identical sequences.

Different types of promoters are known within the art. The present invention refers to a chimeric promoter, wherein different promoters or parts of different promoters, i.e., an oligonucleotide having promoter activity or parts thereof are combined.

In the following, the elements of the present invention will be described in more detail. These elements are listed with specific embodiments, however, it should be understood that they may be combined in any manner and in any number to create additional embodiments. The variously described examples and embodiments should not be construed to limit the present invention to only the explicitly described embodiments. This description should be understood to support and encompass embodiments which combine the explicitly described embodiments with any number of the disclosed elements. Furthermore, any permutations and combinations of all described elements in this application should be considered disclosed by the description of the present application unless the context indicates otherwise.

Throughout this specification and the claims, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example"), provided herein is intended merely to better illustrate the invention and does not pose a limitation on the scope of the invention otherwise claimed. No language in the specification should be construed as indicating any non-claimed element essential to the practice of the invention.

There is a high demand for promoters and combinations of promoters, respectively, which regulate transcription and optionally expression of one or more genes dependent on a specific condition such as varying carbon sources including for example varying ratios of carbon sources. The conditions are for example intracellular and/or extracellular conditions and there is a need for promoters resulting in optimized adaptation of the transcription of genes of a cell to one or more conditions. Such promoters shall allow optimizing transcription in a cell in that transcription of a gene is activated or increased when needed and stopped or decreased/lowered when not needed anymore to optimize the use of cellular resources. Thus, there is a high demand for promoters to optimize transcription of genes in cells and thus, gene expression, which are adequate for control under specific conditions such as a carbon source.

## Summary

The present invention refers to a chimeric promoter characterized in that it comprises two or more oligonucleotide sequence(s) or parts thereof regulating the transcription of a gene of an anabolic and/or a catabolic pathway such as the glycolysis and the gluconeogenesis and increases the transcript level of an RNA typed as messenger RNA fragment encoding for a protein selected from the group consisting of enzymes for example a carbohydrate modifying enzyme, structural proteins, coenzymes, transporters, antibodies, hormones and regulators, as regulatory RNA fragment, as enzymatically active RNA fragment or as transfer RNA fragment, said chimeric promoter having sequence identity to SEQ ID NO.1, SEQ ID NO.2 or SEQ ID NO.3. The carbohydrate modifying enzyme is for example selected from the group consisting of EC 5.1.3, EC 5.3.1, EC 2.7.1, EC 2.2.1, and EC 1.1.1. The transporter is for example selected from the group consisting of TC 2.A.1.1 and 2.A.1.2.

A chimeric promoter of the present invention allows an increase in the transcript level of the RNA fragment for example in a yeast host cell when growing the host cell transformed with at least one recombinant DNA fragment comprising the chimeric promoter on a carbon source for example selected from the group consisting of glucose, xylose, ethanol and a combination thereof.

A chimeric promoter of the present invention comprises for example an increased number of transcription binding factors for example selected from the group consisting of REB1, GCR1, GCR2, PHD1, TYE7, PHO2, PHO4, AZF1 and a combination thereof.

The chimeric promoter of SEQ ID NO.1 (pCHI3) comprises for example SEQ ID NO.10 and SEQ ID NO.6, the chimeric promoter of SEQ ID NO.2 (pCHI4) comprises for example SEQ ID NO.8, SEQ ID NO.7 and SEQ ID NO.11, and the chimeric promoter of SEQ ID NO.3 (pCHI5) comprises for example SEQ ID NO.12, SEQ ID NO.4, SEQ ID NO.5 and SEQ ID NO.9.

The present invention further refers to a recombinant DNA fragment comprising a chimeric promoter of the present invention, to an expression plasmid comprising at least one recombinant DNA fragment, and to a host cell transformed with at least one recombinant DNA fragment or transformed with at least one expression plasmid.

## Detailed Description

The inventors of the present invention have therefore set themselves the task to develop novel and improved chimeric promoter which enables highly specific, reliable transcriptional control of one or more genes of the cell in response to varying intracellular and/or extracellular conditions such as varying carbon sources including varying ratios of carbon sources, wherein the chimeric promoters are highly feasible for industrial applications.

In addition, the chimeric promoters of the present invention extend the collection of promoters for genetic engineering in a microorganism such as yeast for example *Saccharomyces cerevisiae* (*S. cerevisiae*) including for example an increased diversity of expression levels. Promoters of the present invention comprise or consist of heterologous oligonucleotides forming heterologous promoters. As numerous genes are expressed, it is advantageous to have several heterologous promoters, even if they may result in a similar transcript level and may have a similar expression rate, respectively, to increase genetic stability of the engineered microorganism characterized by no or a rare loss of introduced genetic elements by homologous recombination.

The inventors of the present invention surprisingly found that this task can be solved by a chimeric promoter comprising two or more oligonucleotide sequence(s) or parts thereof regulating the transcription of a gene of an anabolic pathway and/or a catabolic pathway, where the catabolic pathway corresponds to the anabolic pathway, such as genes of the glycolysis and of the gluconeogenesis, respectively, which for example increases the transcript level of an RNA (based on the transcription rate and the stability of the RNA) such as a messenger RNA fragment encoding for a protein selected from the group consisting of enzymes, structural proteins, coenzymes, transporters, antibodies, hormones and regulators, as regulatory RNA fragment, as enzymatically active RNA fragment or as transfer RNA fragment, said chimeric promoter having sequence identity to SEQ ID NO.1 (pCHI3), SEQ ID NO.2 (pCHI4) or SEQ ID NO.3 (pCHI5).

The nomenclature of amino acids, peptides, nucleotides and nucleic acids within the present application follows the suggestions of IUPAC. Generally, amino acids are named within this document according to the one letter code.

The "chimeric promoter" is an oligonucleotide having promoter activity. An "oligonucleotide" according to the present invention is to be understood as a single-stranded or double-stranded DNA or RNA molecule comprising from 2 to 1000 nucleic acids, preferably from 10 to 900 nucleic acids, further preferred from 50 to 850 nucleic acids and most preferred from 100 to 820 nucleic acids.

The terms "DNA" and "RNA" are well known to a person skilled in the art. While DNA contains deoxyribose, RNA contains ribose (in deoxyribose there is no hydroxyl group attached to the pentose ring in the 2' position). The complementary base to adenine is not thymine, as it is in DNA, but rather uracil, which is an unmethylated form of thymine.

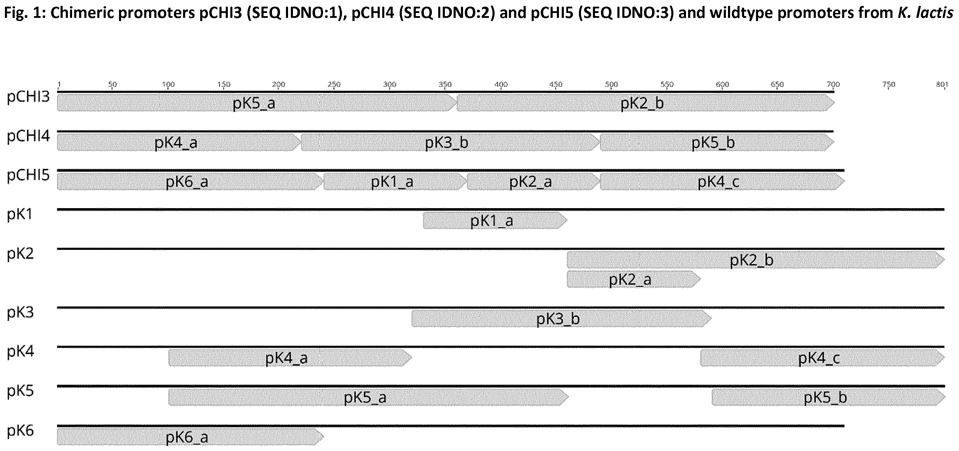

The chimeric promoter of the present invention comprises or consists of two or more (e.g., 2, 3, 4, 5, 6, 7, 8, 9 or 10) oligonucleotide sequence(s) or parts thereof (e.g., a promoter or part thereof) regulating the transcription of one or more genes of the anabolic and catabolic pathway, i.e., genes of opposing metabolic pathways, for example glycolysis (glucose degradation) and the gluconeogenesis (glucose synthesis) (e.g., Fig. 1). The genes are not classified according to the pathway they belong to, but according to their activation under the specific condition(s) of the pathway for example the presence of a high or low glucose level for example in *5. cerevisiae*.

According to this classification genes of glycolysis are for example PGK1, ENO1 or PFK2, and genes of gluconeogenesis are for example PGI1, TPI1 or FBA1.

The chimeric promoters pCHI3, pCHI4 and pCHI5 have the following sequences:

| SEQ ID NO.1 (pCHI3): |
| --- |
| AAATGATCACAAATGTGATTGATGATTTGACACGACTAGAAAAGAGAACGAAAAAGGGAAAT TCCATGTCACGTGCGTTGGCACGTGACATGGAATATCGAAGAAAGAAAAAAAAAAACGATCT CGTCCTAGTGGAAGCCCAGAGTCTGGTCCCCCCGGAGTCTTCCCAAAACAAGAAGCTGACACA TGTTGACACAGAACACCCCACAGCAAATGCACCACGCTACGTAGATCAGGAAGCTTAACTCTA GCGACCTGTCGCTCGCCCCACAGAACCTCACCCGAGAACCACACATTACACGCCGCCAGCTCC CACTATACTCATCTTGCTTCCCTTAAGCGTTCTCACGATTCGTTCGCTGGATAATTATCTTCTTCC GTCTATCTTCTTCTAAATTGATCAGTATCATAATATGGAAAAAAGGTTGTTCGTGAATTTTTCC TCCATTCAATTCATATATAATAAGAACAATGAATTGCATACTTCCCAAATCTTGATAATTCGTTT TAATATCTATCAGTAGTTGTTTATATTCAATTATATCTTGTGTATACAAAACCATCATCCAAAAA CCAATCCAAGTTTCGTTTGACTTATAACCAATAGTACCAAAAATTAAACCTTAATTTTGAAGTAC TTTATAATTTAAACTTAATACGAATTAAACCAAAAAACCCACAACACGTAAAAGAAATTACA |
| **SEQ ID NO.2 (pCHI4):** |
| TTACTTTCCCGGATAATTTCGAAGAGGATATTACCCGCTTACGCCAGATGCATGCGAACTTGAT GCAGCACATGAAGAGACAATCAACAGAGACCCCAGGAAATCTTGAAGAACAACAGAAACACA TCAATGATATCGTAGATACCATTGAGAGATATAACTGAATAAATACTTCAAATCACGTGATGA ATCACGTGCCACAATTACCCTGACTTTTTGTTTACGCAGCAAACATGCAGCATCCACTAACTTC AGAGATTCCTGTAAGATAAGTGGTTCGTTATTTTCCGGATTCCAATTTTGGTGGTGCTCCGAAA AGTGGAAGCTCGTGATCCTACATGCTCACGAAACCCATCATCATGCAATCCACATTAAAGGAA GGGAAAAGGATATTGAACTTTTGACTATTTAGTATAAATGAAAACTACTTTGTAAGCTTGGAC AGAGGAATAATTTCTGATTCGTGCTTCTGCTTCTACTGACTTGCAAATTTACATATAAATATAC GTCAAAAGGGGATTCATTAATTAGAAAATTCTCTTTTTCAATAGTTGCTATTCATTATCAATCTA TTCAACTCAATTGGTTATTATTTTCATCTTTTTGTCATCCTAAACCATCAACAATATTTAAATATA TCTGTTGCTACATTAAGAGTTACTTCAGAAATAACAAAAAAATCGATCAAGAATTAATAAAA |
| **SEQ ID NO.3 (pCHI5):** |

(continued)

```
TAATATTTATCTGGATAATTGTGAGTGCTTGAATTACCTTCTATCTCACGTGATTTGATTCTATT
GGACAAAAAGGGAAAGACCCTCCTGAGAGATTAAAATAATTACCCGGATCTAAGAGATTTCA
CGTTAAAATATACATCTCCTTCCGAGTACTCTTAGCTTCCACTATTTTAACGGGGCTATTCATGC
GTTCCGGGTAATGAGGTGTTCCCGGAGCCAAAAATCATCTTCCTTTATCAGAAAGACACGTTC
ACAATCCAGGCACCCCACAGAGAAAAAAAAAAGAAGAAGCCCGGAAGCTGGCACGCCATCAT
CAACCACCGCTCGGTTTACACGCATCCCAACTGTCTTTTTTTTCTGGAATCCTGGATAATTATCT
TCTTCCGTCTATCTTCTTCTAAATTGATCAGTATCATAATATGGAAAAAAGGTTGTTCGTGAAT
TTTTCCTCCATTCAATTCATATATAATAAGAACAATGAATTGCTCTTTATATATATATTCTCTG
GTGAAGGTTCTTGATCAATTGCTTCTTCCAATTGGTATTTCGATTGTATTCTTGAGTCACGTAGA
AAGGAATTTGTGATTAGGGTTTAATCGCTGAATTCTGAAGTGACCTTTTAACTGACCGTAAAG
TACAATAAAAGGTTTTAAAGTTCTGTTAAGATAGATTCTAAAACAGAAATAAAAAGACAAATA
TCAGAA
```

The chimeric promoters of the present invention comprise or consist of oligonucleotides (e.g., parts of promoters) selected from the group consisting of pK1a (SEQ ID NO.4), pK2a (SEQ ID NO.5), pK2b (SEQ ID NO.6), pK3b (SEQ ID NO.7), pK4a (SEQ ID NO.8), pK4c (SEQ ID NO.9), pK5a (SEQ ID NO.10), pK5b (SEQ ID NO.11), pK6a (SEQ ID NO.12), and combinations thereof.

The sequences of these parts are shown in the following:

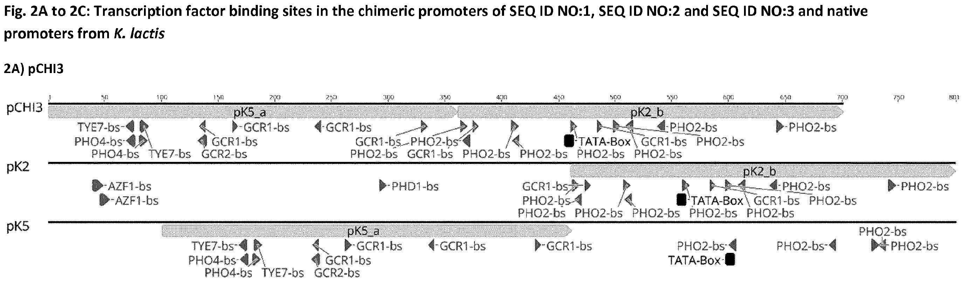

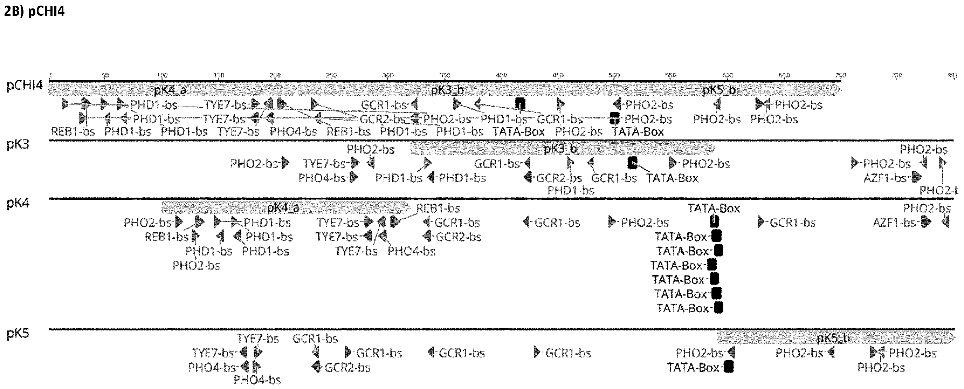

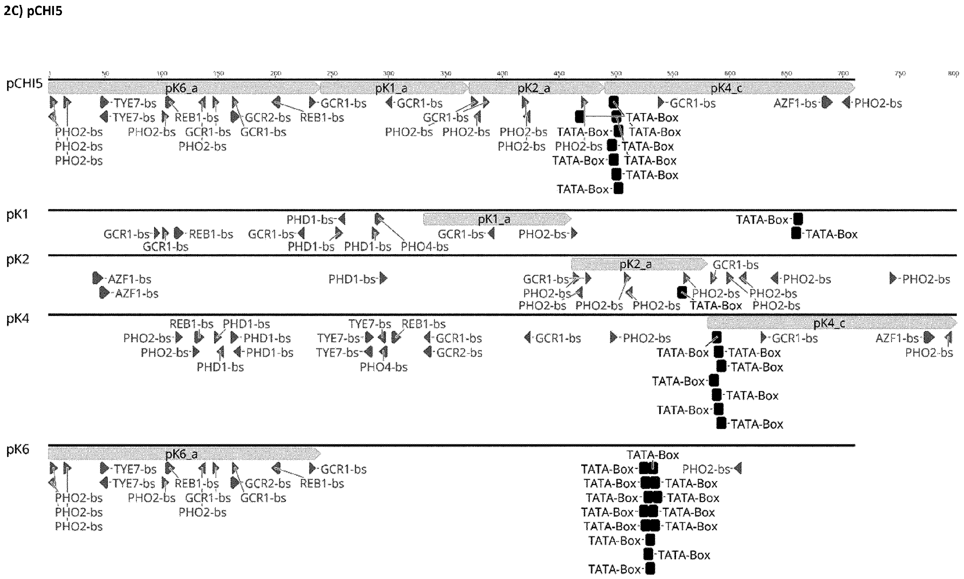

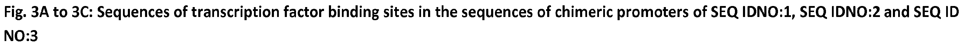

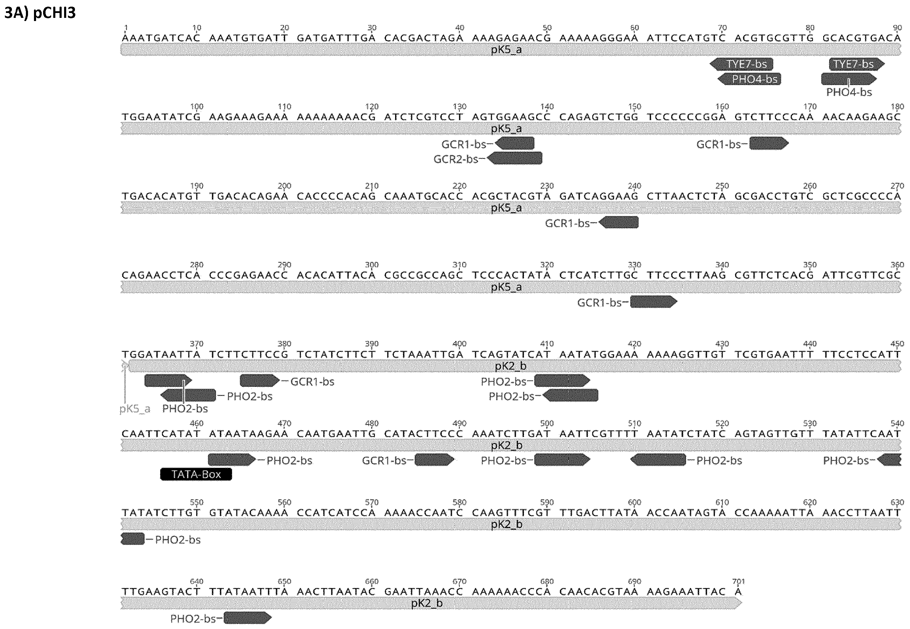

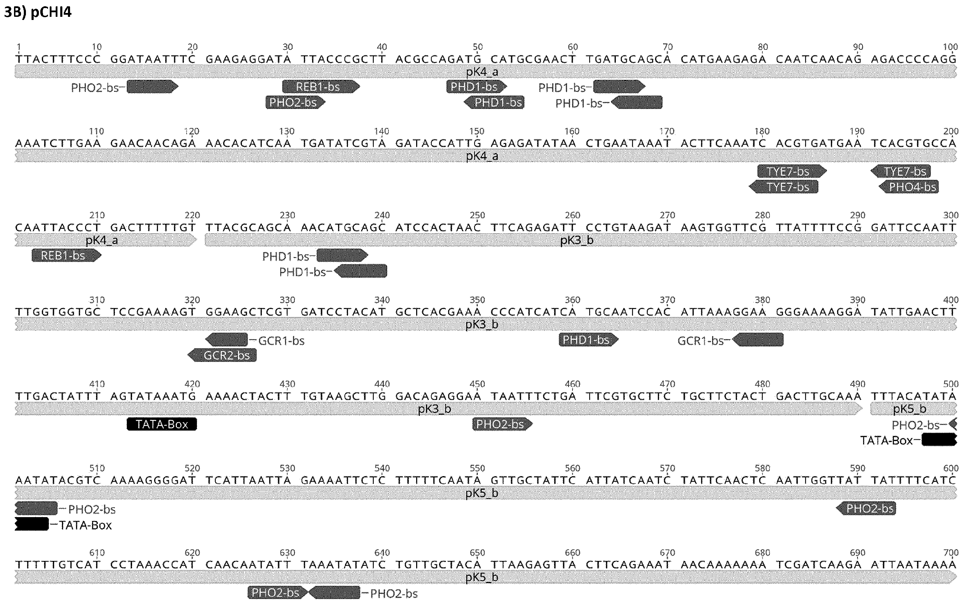

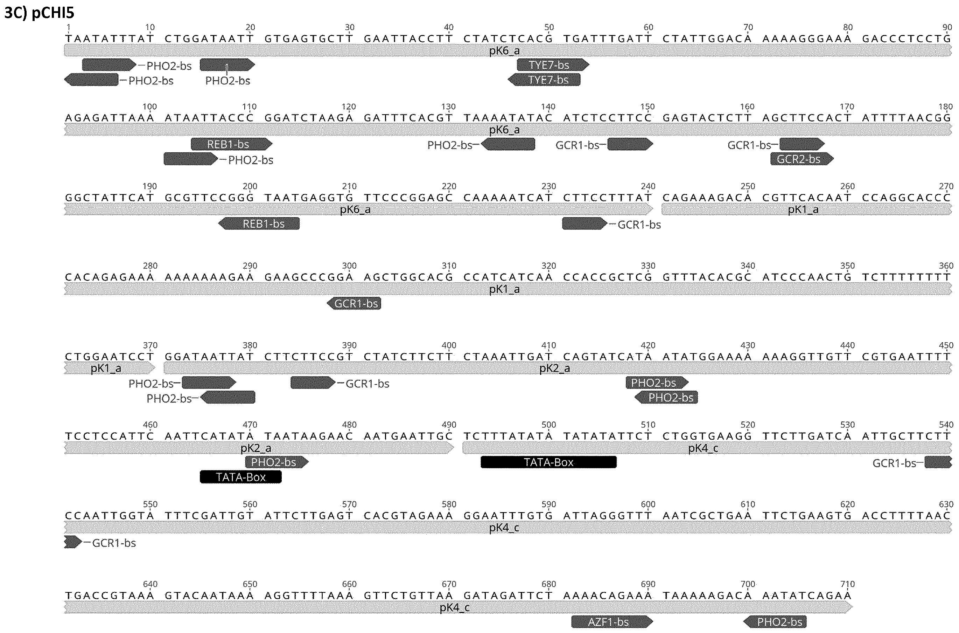

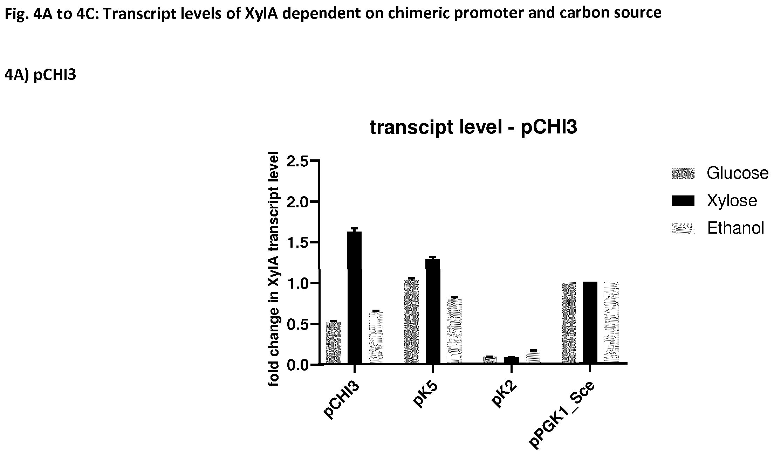

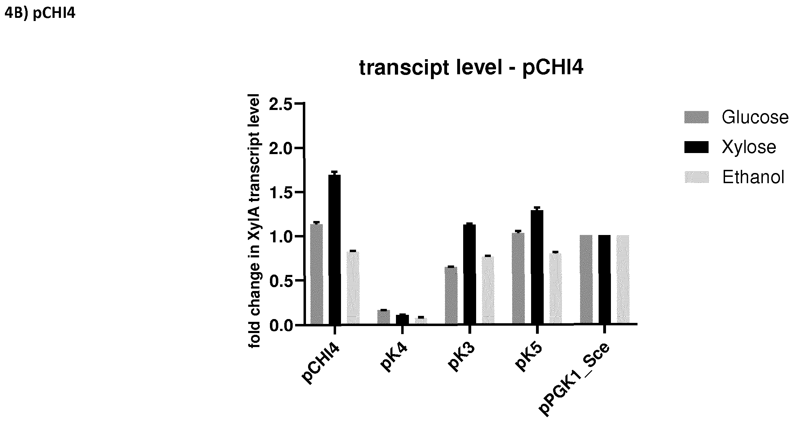

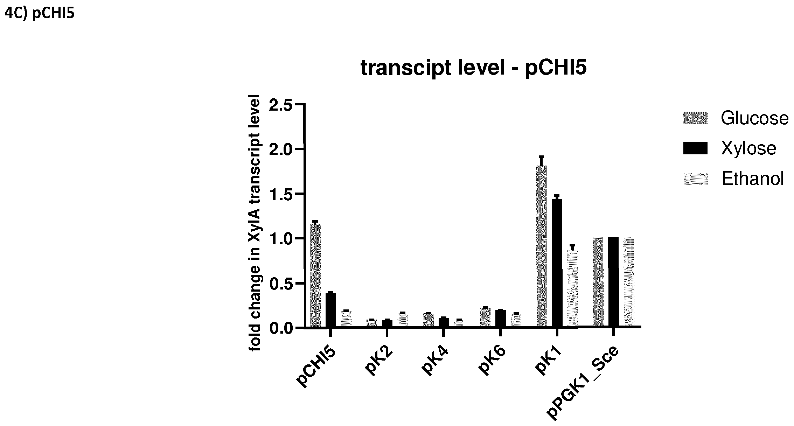

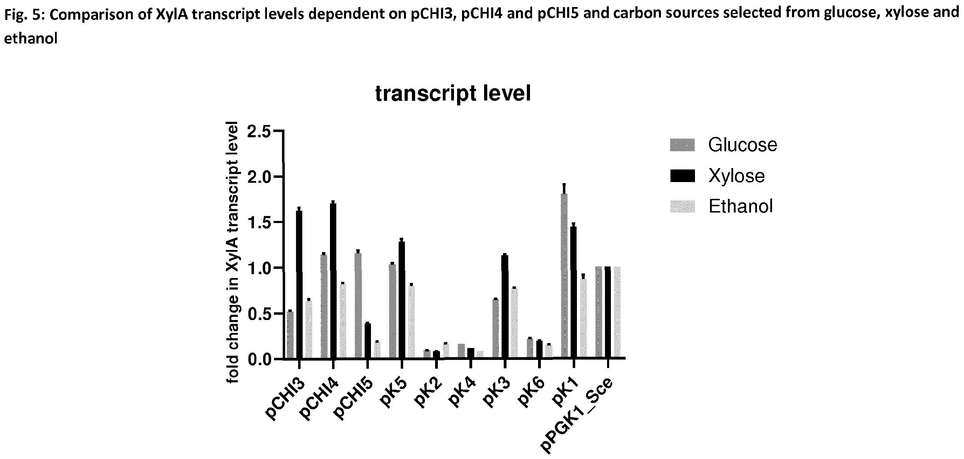

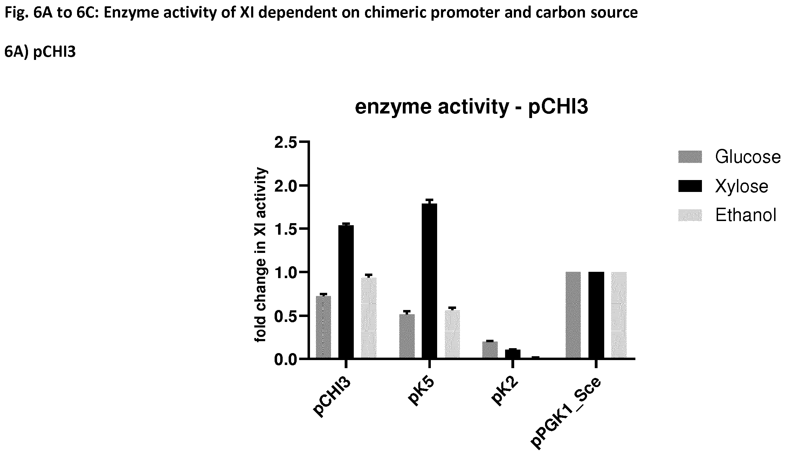

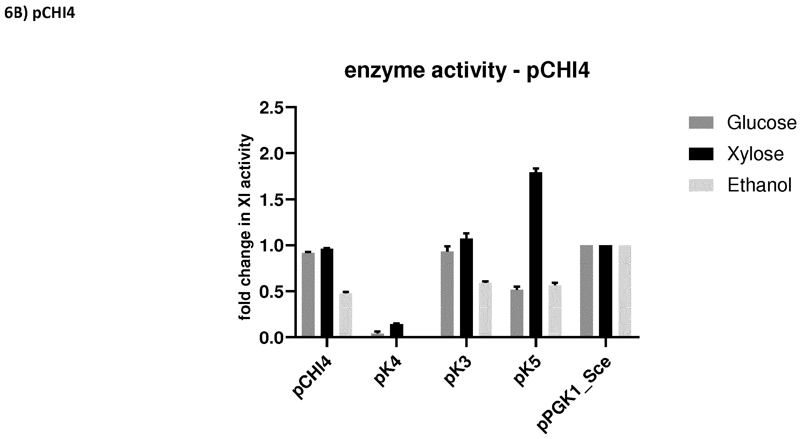

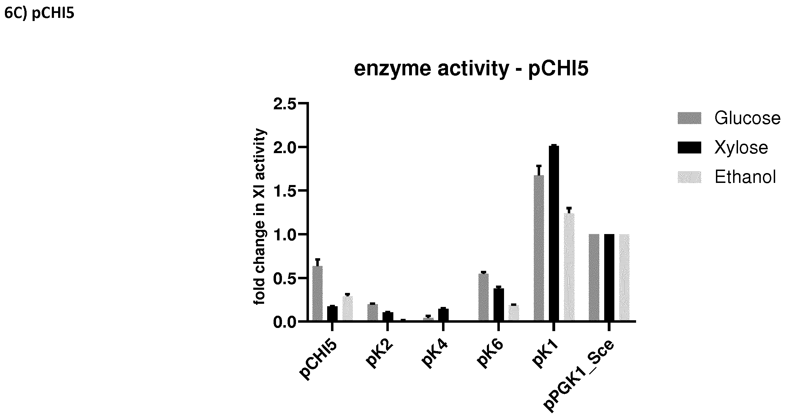

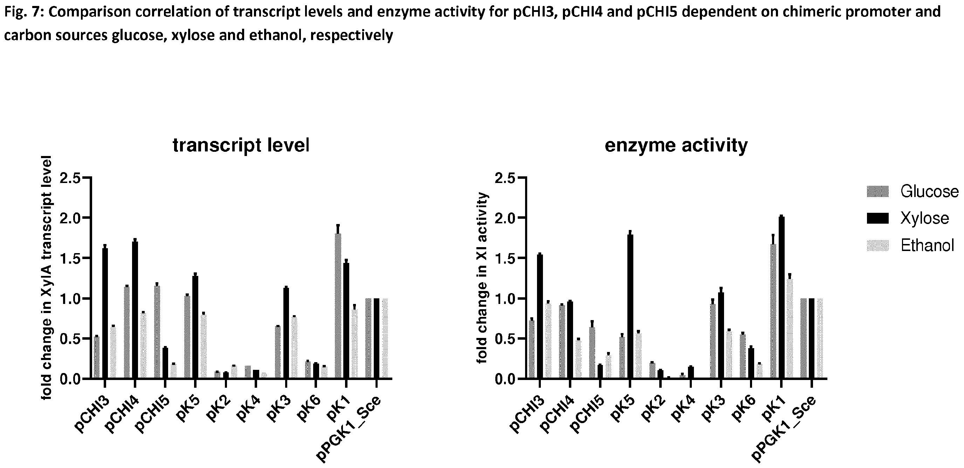

| SEQ ID NO.4 | CAGAAAGACACGTTCACAATCCAGGCACCCCACAGAGAAAAAAAAAAGAAGAAGCC CGGAAGCTGGCACGCCATCATCAACCACCGCTCGGTTTACACGCATCCCAACTGTCTT TTTTTTCTGGAATCCT |
|---|---|
| SEQ ID NO.5 | GGATAATTATCTTCTTCCGTCTATCTTCTTCTAAATTGATCAGTATCATAATATGGAAA AAAGGTTGTTCGTGAATTTTTCCTCCATTCAATTCATATATAATAAGAACAATGAATT GC |
| SEQ ID NO.6 | GGATAATTATCTTCTTCCGTCTATCTTCTTCTAAATTGATCAGTATCATAATATGGAAA AAAGGTTGTTCGTGAATTTTTCCTCCATTCAATTCATATATAATAAGAACAATGAATT GCATACTTCCCAAATCTTGATAATTCGTTTAATATCTATCAGTAGTTGTTTATATTCAA TTATATCTTGTGTATACAAAACCATCATCCAAAAACCAATCCAAGTTTCGTTTGACTTA TAACCAATAGTACCAAAAATTAAACCTTAATTTTGAAGTACTTTATAATTTAAACTTAA TACGAATTAAACCAAAAAACCCACAACACGTAAAAGAAATTACA |
| SEQ ID NO.7 | TTACGCAGCAAACATGCAGCATCCACTAACTTCAGAGATTCCTGTAAGATAAGTGGTT CGTTATTTTCCGGATTCCAATTTTGGTGGTGCTCCGAAAAGTGGAAGCTCGTGATCCT ACATGCTCACGAAACCCATCATCATGCAATCCACATTAAAGGAAGGGAAAAGGATAT TGAACTTTTGACTATTTAGTATAAATGAAACTACTTTGTAAGCTTGGACAGAGGAAT AATTTCTGATTCGTGCTTCTGCTTCTACTGACTTGCAAA |
| SEQ ID NO.8 | TTACTTTCCCGGATAATTTCGAAGAGGATATTACCCGCTTACGCCAGATGCATGCGAA CTTGATGCAGCACATGAAGAGACAATCAACAGAGACCCCAGGAAATCTTGAAGAACA ACAGAAACACATCAATGATATCGTAGATACCATTGAGAGATATAACTGAATAAATACT TCAAATCACGTGATGAATCACGTGCCACAATTACCCTGACTTTTTGT |
```

(continued)

| SEQ ID NO.9 | TCTTTATATATATATATTCTCTGGTGAAGGTTCTTGATCAATTGCTTCTTCCAATTGGTA<br>TTTCGATTGTATTCTTGAGTCACGTAGAAAGGAATTTGTGATTAGGGTTTAATCGCTG<br>AATTCTGAAGTGACCTTTTAACTGACCGTAAAGTACAATAAAAGGTTTTAAAGTTCTG<br>TTAAGATAGATTCTAAAACAGAAATAAAAAGACAAATATCAGAA |
|---|---|
| SEQ ID NO.10 | AAATGATCACAAATGTGATTGATGATTTGACACGACTAGAAAAGAGAACGAAAAAG<br>GGAAATTCCATGTCACGTGCGTTGGCACGTGACATGGAATATCGAAGAAAGAAAAAA<br>AAAAACGATCTCGTCCTAGTGGAAGCCCAGAGTCTGGTCCCCCCGGAGTCTTCCCAA<br>AACAAGAAGCTGACACATGTTGACACAGAACACCCCACAGCAAATGCACCACGCTAC<br>GTAGATCAGGAAGCTTAACTCTAGCGACCTGTCGCTCGCCCCACAGAACCTCACCCGA<br>GAACCACACATTACACGCCGCCAGCTCCCACTATACTCATCTTGCTTCCCTTAAGCGTT<br>CTCACGATTCGTTCGCT |
| SEQ ID NO.11 | TTTACATATAAATATACGTCAAAAGGGGATTCATTAATTAGAAAATTCTCTTTTTCAAT<br>AGTTGCTATTCATTATCAATCTATTCAACTCAATTGGTTATTATTTTCATCTTTTTGTCAT<br>CCTAAACCATCAACAATATTTAAATATATCTGTTGCTACATTAAGAGTTACTTCAGAAA<br>TAACAAAAAAATCGATCAAGAATTAATAAAA |
| SEQ ID NO.12 | TAATATTTATCTGGATAATTGTGAGTGCTTGAATTACCTTCTATCTCACGTGATTTGAT<br>TCTATTGGACAAAAAGGGAAAGACCCTCCTGAGAGATTAAAATAATTACCCGGATCT<br>AAGAGATTTCACGTTAAAATATACATCTCCTTCCGAGTACTCTTAGCTTCCACTATTTT<br>AACGGGGCTATTCATGCGTTCCGGGTAATGAGGTGTTCCCGGAGCCAAAAATCATCT<br>TCCTTTAT |

The chimeric promoter pCHI3 (SEQ ID NO.1) comprises or consists of the oligonucleotides (e.g., parts of the promoters) pK5a (SEQ ID NO.10) and pK2b (SEQ ID NO.6). The chimeric promoter pCHI4 (SEQ ID NO.2) comprises or consists of oligonucleotides (e.g., parts of the promoters) pK4a (SEQ ID NO.8), pK3b (SEQ ID NO.7) and pK5b (SEQ ID NO.11). The chimeric promoter pCHI5 (SEQ ID NO.3) comprises or consists of oligonucleotides (e.g., parts of the promoters) pK6a (SEQ ID NO.12), pK1a (SEQ ID NO.4), pK2a (SEQ ID NO.5) and pK4c (SEQ ID NO.9). The chimeric promoters and their parts (oligonucleotides) are shown in Fig. 1.

In addition, in the chimeric promoters of the present invention transcription factor binding sites are enriched which are for example selected from the group consisting of REB1 (e.g., having the sequence RTTACCCK), GCR1 (e.g., having the sequence CTTCC), GCR2 (e.g., having the sequence GCTTCCA), PHD1 (e.g., having the sequence SMTGCA), TYE7 (e.g., having the sequence CACGTGA), PHO2 (e.g., having the sequence ATAWTW), PHO4 (e.g., having the sequence GCRCGYG), AZF1 (e.g., having the sequence AAAMRGMAA) and combinations thereof (Fig. 2 and 3), wherein R, K, W, M, Y etc. are specified according to IUPAC for example R = A or G, K = G or T, W = A or T, M = A or C and Y = C or T. The chimeric promoter of the present invention comprises a "core region" comprising at least 210 bp at the 5'-end of the chimeric promoter being closely located to the starting point of the translation and being unmodified, i.e., the core region corresponds to the sequence of a native oligonucleotide. The native oligonucleotide according to the present invention is a nucleic acid sequence which is identical to a sequence found in the microorganism where it originates from. For example an oligonucleotide originating from *K. lactis* being transferred to a host microorganism such as *S. cerevisiae* comprises or consists of a core region corresponding to a sequence of *K. lactis*. The oligonucleotide(s) or parts thereof forming the chimeric promoter of the present invention is/are for example oriented in the same direction and is/are located at the same position as in the oligonucleotide which it is/they are originating from.

A chimeric promoter of the present invention, i.e., an oligonucleotide having promoter activity, is either transferred to a host cell which is a different microorganism than the microorganism, where the oligonucleotide(s) of the promoter originate from or it is the same microorganism, where the oligonucleotide(s) of the promoter originate from.

The chimeric promoter according to the present invention comprises a nucleic acid sequence having sequence identity to SEQ ID NO.1, SEQ ID NO.2 or SEQ ID NO.3.

In another example the nucleic acid sequence is selected from the group consisting of SEQ ID NO.1, SEQ ID NO.2 or SEQ ID NO.3.

The chimeric promoter according to the present invention increases the transcript level of certain RNA fragments which are for example functionally linked to the chimeric promoter, i.e., controlled by the chimeric promoter, for example dependent on changing conditions such as different carbon sources including varying ratios of carbon sources. A chimeric promoter of the present invention has a specific functional characteristic, i.e., it leads to an increased transcript level of a certain RNA when the host is grown on and the promoter is exposed to a specific carbon source, respectively, and an unchanged or decreased transcript level of a certain RNA when the host is grown on and the promoter is exposed to another specific carbon source, respectively, compared to the respective transcript level for example resulting from a promoter of the state of the art. Oligonucleotides forming the chimeric promoter are selected and combined to (specifically) regulate the transcript levels. An oligonucleotide of the chimeric promoter alone may show a different transcript level than the combination of oligonucleotides forming the chimeric promoter. The great advantage of the present invention is the combination of oligonucleotides resulting in the chimeric promoter specifically regulating one or more transcript levels for example dependent on the carbon source.

The term "increase" or "decrease of the transcript level" is thereby to be understood for example as an increase or decrease compared to the transcript level resulting from an oligonucleotide of the state of the art which is an oligonucleotide known in the prior art natively or recombinant present in a microorganism. For example if the oligonucleotide having promoter activity originates from *K. lactis* and is transferred to *5. cerevisiae,* the reference to determine the increase or decrease of a transcript level is an oligonucleotide having promoter activity in *K. lactis* or *5. cerevisiae* which is for example natively or recombinant present in this microorganism. For example the transcript level of an RNA based on the activity of a chimeric promoter of the present invention such as pCHI3, pCHI4 or pCHI5 is determined in comparison to a native promoter of *5. cerevisiae* e.g., pPKG1_Sce which represents in this case the oligonucleotide of the state of the art. The "increase or decrease of the transcript level" is generally to be determined as follows:

$$\frac{RTL_I}{RTL_S}$$

$RTL_I$ - relative transcript level of a reporter system (e.g., XylA, SEQ ID NO.13) controlled by a chimeric promoter according to the invention

$RTL_S$ - relative transcript level of a reporter system (e.g., XylA, SEQ ID NO.13) controlled by an oligonucleotide according to the state of the art

Thereby the relative transcript level is measured as the concentration of RNA of the reporter system in a cell extract in relation to the concentration of the RNA of a housekeeping gene (e.g., ACT1) in the same cell extract.

Whereas $RTL_I$ and RTLs are determined by use of the same type of host cell whereas the host cell is transformed with at least one recombinant DNA fragment comprising the respective chimeric promoter and the host cell is grown under identical state of the art conditions whereas the host cell is harvested within the exponential growth phase.

Within a preferred embodiment the transcript level of a specific gene is increased when growing a yeast host cell, preferably *5. cerevisiae,* transformed with at least one recombinant DNA fragment comprising a chimeric promoter according to the present invention on a carbon source including varying ratios of carbon sources for example selected from the group consisting of glucose, mannose, fructose, galactose, xylose, arabinose, sucrose, trehalose, raffinose, glycerol, ethanol, acetate and lactate, in particular glucose, xylose and/or ethanol. The increase was determined as follows:

$$\frac{RTL_{Ie}}{RTL_{Se}}$$

$RTL_{Ie}$ - relative transcript level of the messenger RNA encoding for SEQ ID NO.15 controlled by the chimeric promoter of SEQ ID NO.1, SEQ ID NO.2, or SEQ ID NO.3.

$RTL_{Se}$ - relative transcript level of the messenger RNA encoding for SEQ ID NO.15 controlled by the oligonucleotide SEQ ID NO.14

Thereby the relative transcript level is measured as the concentration of messenger RNA encoding for SEQ ID NO.15 in a yeast (*S. cerevisiae*) cell extract in relation to the concentration of the messenger RNA of the housekeeping gene encoding for actin in the same yeast cell extract.

Whereas $RTL_{Ie}$ and $RTL_{Se}$ are determined by use of the same type of yeast host cell (*S. cerevisiae*) whereas the yeast host cell is transformed with at least one recombinant DNA fragment comprising the respective chimeric promoter and the yeast host cell is grown under identical state of the art conditions whereas the yeast host cell is harvested within the exponential growth phase.

Within a particularly preferred embodiment of the present invention, the transcript level of the gene in a yeast host cell transformed with at least one recombinant DNA fragment comprising the chimeric promoter according to the present invention is increased depending on different conditions such as different carbon sources including varying ratios of carbon sources for example in a range of 1.1-fold to 10-fold, 1.2-fold to 9-fold, 1.3-fold to 8-fold, 1.4-fold to 7-fold, 1.5-fold to 6-fold, 1.4-fold to 5-fold, 1.5-fold to 4-fold, 1.6-fold to 3-fold, 1.7-fold to 2.5-fold, 1.8-fold to 2.4-fold, 1.9-fold to 2.3-fold, or by at least 1.1-fold, at least 1.2-fold, at least 1.3-fold, at least 1.4-fold by at least 1.5-fold, at least 1.6-fold, at least 1.7-fold, at least 1.8-fold, at least 1.9-fold, at least 2-fold, at least 2.5-fold, at least 3-fold, at least 3.5-fold, at least 4-fold , at least 4.5-fold, at least 5-fold or more, or by 1.1-fold, 1.2-fold, 1.3-fold, 1.4-fold, 1.5-fold, 1.6-fold, 1.7-fold, 1.8-fold, 1.9-fold, 2-fold, 2.1-fold, 2.2-fold, 2.3-fold, 2.4-fold, 2.5-fold, 2.6-fold, 2.7-fold, 2.8-fold, 2.9-fold, 3-fold, 3.1-fold, 3.2-fold, 3.3-fold, 3.4-fold or 3.5-fold when growing the host cell such as yeast on a carbon source selected from the group consisting of glucose, mannose, fructose, galactose, xylose, arabinose, sucrose, trehalose, raffinose, glycerol, ethanol, acetate, lactate and combinations thereof; or selected from the group consisting of glucose, mannose, fructose, xylose, sucrose, glycerol, ethanol and combinations thereof; or selected from the group consisting of glucose, mannose, glycerol, ethanol, xylose and combinations thereof; or selected from the group consisting of glucose, xylose, ethanol and combinations thereof.

Optionally in addition, the chimeric promoter according to the present invention increases the enzyme activity of an enzyme encoded by an RNA controlled by the oligonucleotide depending on different conditions such as a different carbon source including varying ratios of carbon sources. The term "x-fold enzyme activity" is thereby to be understood as an increase or decrease of the enzyme activity compared to the enzyme activity of an oligonucleotide with promoter activity of the state of the art. The "x-fold enzyme activity" is generally to be determined as follows:

$$\frac{EA_I}{EA_S}$$

$EA_I$ - enzyme activity of a reporter system (e.g., XI, SEQ ID NO.15) controlled by a chimeric promoter according to the invention

$EA_S$ - enzyme activity of a reporter system (e.g., XI, SEQ ID NO.15) controlled by an oligonucleotide according to the state of the art

Thereby the enzyme activity is measured as the amount of a substrate converted per minute by defined amount of a cell extract excluding the background activity of the reporter system.

Whereas $EA_I$ and $EA_S$ are determined by use of the same type of host cell whereas the host cell is transformed with at least one recombinant DNA fragment comprising the respective chimeric promoter and the host cell is grown under identical state of the art conditions whereas the host cell is harvested within the exponential growth phase.

Within a preferred embodiment the enzyme activity is increased dependent on different conditions such as different carbon sources including varying ratios of carbon sources for example in a range of 1.1-fold to 10-fold, 1.2-fold to 9-fold, 1.3-fold to 8-fold, 1.4-fold to 7-fold, 1.5-fold to 6-fold, 1.4-fold to 5-fold, 1.5-fold to 4-fold, 1.6-fold to 3-fold, 1.7-fold to 2.5-fold, 1.8-fold to 2.4-fold, 1.9-fold to 2.3-fold, or by at least 1.5-fold or at least 2-fold or 1.1-fold to 5-fold, 1.2-fold to 4-fold, 1.3-fold to 3.5-fold, 1.4-fold to 3-fold, 1.5-fold to 2.9-fold, 1.6-fold to 2.8-fold, 1.7-fold to 2.7-fold, 1.8-fold to 2.6-fold, 1.9-fold to 2.5-fold or 2-fold when growing a host cell, such as yeast for example *5. cerevisiae,* transformed with at least one recombinant DNA fragment comprising a chimeric promoter according to the present invention on a carbon source selected from the group consisting of glucose, mannose, fructose, galactose, xylose, arabinose, sucrose, trehalose, raffinose, glycerol, ethanol, acetate, lactate and combinations thereof; or selected from the group consisting of glucose, xylose, ethanol and combinations thereof.

The increase was determined as follows:

$$\frac{EA_{Ie}}{EA_{Se}}$$

$EA_{Ie}$ - enzyme activity of the protein SEQ ID NO.15 controlled by the chimeric promoter SEQ ID NO.1, SEQ ID NO.2 or SEQ ID NO.3.

$EA_{Se}$ - enzyme activity of the protein SEQ ID NO.15 controlled by an oligonucleotide SEQ ID NO.14.

Thereby the enzyme activity is measured as the amount of xylose converted per minute by defined amount of a cell extract excluding the background activity of the reporter system.

Whereas $EA_{le}$ and $EA_{Se}$ are determined by use of the same type of host cell (*S. cerevisiae*) whereas the host cell is transformed with at least one recombinant DNA fragment comprising the respective chimeric promoter and the host cell is grown under identical state of the art conditions whereas the host cell is harvested within the exponential growth phase.

Within an embodiment of the present invention, one or more enzyme activity(ies) in a yeast host cell transformed with at least one recombinant DNA fragment comprising the chimeric promoter according to the present invention is increased and one or more other enzyme activity(ies) remain unchanged or decrease dependent on different conditions such as different carbon sources including varying ratios of carbon sources. The increase or decrease of the transcript level is for example in a range of 1.1-fold to 10-fold, 1.2-fold to 9-fold, 1.3-fold to 8-fold, 1.4-fold to 7-fold, 1.5-fold to 6-fold, 1.4-fold to 5-fold, 1.5-fold to 4-fold, 1.6-fold to 3-fold, 1.7-fold to 2.5-fold, 1.8-fold to 2.4-fold, 1.9-fold to 2.3-fold, or by at least 1.1-fold, at least 1.2-fold, at least 1.3-fold, at least 1.4-fold by at least 1.5-fold, at least 1.6-fold, at least 1.7-fold, at least 1.8-fold, at least 1.9-fold, at least 2-fold, at least 2.5-fold, at least 3-fold, at least 3.5-fold, at least 4-fold , at least 4.5-fold, at least 5-fold or more or by 1.1-fold, 1.2-fold, 1.3-fold, 1.4-fold, 1.5-fold, 1.6-fold, 1.7-fold, 1.8-fold, 1.9-fold, 2-fold, 2.1-fold, 2.2-fold, 2.3-fold, 2.4-fold, 2.5-fold, 2.6-fold, 2.7-fold, 2.8-fold, 2.9-fold, 3-fold, 3.1-fold, 3.2-fold, 3.3-fold, 3.4-fold or 3.5-fold when growing the host cell such as yeast on a carbon source selected from the group consisting of glucose, mannose, fructose, galactose, xylose, arabinose, sucrose, trehalose, raffinose, glycerol, ethanol, acetate, lactate and combinations thereof; or selected from the group consisting of glucose, mannose, fructose, xylose, sucrose, glycerol, ethanol and combinations thereof; or selected from the group consisting of glucose, mannose, glycerol, ethanol, xylose and combinations thereof; or selected from the group consisting of glucose, xylose, ethanol and combinations thereof.

Within the present invention, the term "regulatory RNA fragment" is to be understood as an RNA chain that has the ability to downregulate a gene expression by being complementary to a part of an mRNA or a gene's DNA. Examples of "regulatory RNA fragments" are MicroRNAs (miRNA) which act through RNA interference (RNAi), where an effector complex of miRNA and enzymes can cleave complementary mRNA, block the mRNA from being translated, or accelerate its degradation. An mRNA may contain regulatory elements itself, such as riboswitches, in the 5' untranslated region or 3' untranslated region; these cis-regulatory elements regulate the activity of that mRNA.

The untranslated regions can also contain elements that regulate other genes.

Within the present invention, the term "enzymatically active RNA fragment" is to be understood as RNA which is part of a protein complex which can catalyze enzymatic reactions within the cell like ribosomal RNA or RNA that forms a catalytically active complex itself such as ribozyme (ribonucleic acid enzymes).

Within the present invention, the term "enzymatically active RNA fragment" is to be understood as RNA which is part of a protein complex which can catalyze enzymatic reactions within the cell like ribosomal RNA or RNA that forms a catalytically active complex itself such as ribozyme (ribonucleic acid enzymes).

Within the present invention, the term "transfer RNA fragment" (tRNA fragment) is to be understood as a small RNA chain of about 80 nucleotides that has the ability to transfer a specific amino acid to a growing polypeptide chain at the ribosomal site of protein synthesis during translation. It has sites for amino acid attachment and an anticodon region for codon recognition that binds to a specific sequence on the messenger RNA chain through hydrogen bonding.

Within the present invention, the term "messenger RNA fragment" (mRNA fragment) is to be understood as a small RNA chain that has the ability to carry information about a protein sequence to the ribosomes. Every three nucleotides (a codon) correspond to one amino acid. In eukaryotic cells, once precursor mRNA (pre-mRNA) has been transcribed from DNA, it is processed to mature mRNA. This removes its introns-noncoding sections of the pre-mRNA. The mRNA is then exported from the nucleus to the cytoplasm, where it is bound to ribosomes and translated into its corresponding protein form with the help of tRNA. In prokaryotic cells, which do not have a nucleus and cytoplasm compartments, mRNA can bind to ribosomes while it is being transcribed from DNA. After a certain amount of time the messenger RNA degrades into its component nucleotides with the assistance of ribonucleases.

Within the present invention the term "structural proteins" refers to proteins which confer stiffness and rigidity to otherwise-fluid biological components. Preferred structural proteins are selected from the group consisting of fibrous proteins such as collagen, elastin and keratin; and globular proteins such as actin and tubulin. Other proteins that serve structural functions and which are to be understood as "structural proteins" within the present invention are motor proteins such as myosin, kinesin, and dynein, which are capable of generating mechanical forces.

Preferred RNA fragments encoding for a structural protein are selected from the group consisting of actine, elastin, filamine, collagen, myosine, lamine.

Preferred RNA fragments encoding for a coenzyme are selected from the group of RNA fragments encoding for polypeptides which are post-translationally modified.

Examples are tryptophan tryptophylquinone (TTQ) and 4-methylidene-imidazole-5-one (MIO).

Preferred RNA fragments encoding for an antibody are selected from the group of RNA fragments encoding for IgA, IgD, IgE, EgG, IgM, IgY and IgW.

Preferred RNA fragments encoding for a hormone are selected from the group of RNA fragments encoding for small peptide hormones such as TRH and vasopressin; insulin; growth hormone; glycoprotein hormones such as luteinizing hormone, follicle-stimulating hormone and thyroid-stimulating hormone.

Preferred RNA fragments encoding for a regulator are selected from the group of RNA fragments encoding for receptors, transcription factors, metabolic sensors, light sensors, electro sensors, mechanical sensors and signal transducers.

Preferred RNA fragments encoding for an enzyme are selected from the group of RNA fragments encoding for carbohydrate-modifying enzymes. Within the present invention, the term "carbohydrate-modifying enzyme" is to be understood as comprising any enzyme capable of modifying any kind of carbohydrate such as (but not limited to) carbohydrate-cleaving, carbohydrate-oxidizing, carbohydrate-reducing, carbohydrate-decarboxylating, carbohydrate-deacetylating, carbohydrate-acetylating, carbohydrate-methylating, carbohydrate-demethylating, carbohydrate-aminating, carbohydrate-phosphorylating, carbohydrate-dephosphorylating, carbohydrate-isomerizing, carbohydrate-epimerizing and carbohydrate-deaminating enzymes.

Within a particularly preferred embodiment of the present invention, the carbohydrate-modifying enzyme is selected from the group consisting of the classes EC 5.1.3, EC 5.3.1, EC 2.7.1, EC 2.2.1, and EC 1.1.1, preferably selected from the group consisting of EC 5.1.3.3, EC 5.3.1.5, EC 2.7.1.17, EC 2.2.1.2, EC 2.2.1.1, EC 1.1.1.1, EC 5.3.1.4, EC 2.7.1.16 and EC 5.1.3.4 as well as mutated enzymes (e.g., comprising substitution, deletion and/or insertions) or fragments thereof.

According to the present invention for example 1 to 80 nucleotides of the oligonucleotide of the present invention are "mutated". Within the present invention the term "mutated" is to be understood as "substituted", "deleted" or "inserted". The term "mutation" is to be understood as "substitution", "deletion" or "insertion". Substitutions are classified as transitions where a purine is exchanged by a purine (A <-> G) or a pyrimidine by a pyrimidine (C <->T) or transversions where a purine is exchanged by a pyridine and vice versa (C/T <-> A/G). Insertions add one or more additional nucleotides (A, C, T or G) into an oligonucleotide. The removal of one or more nucleotides from the DNA is called deletion.

Within a further embodiment, the present invention provides a recombinant DNA fragment comprising the oligonucleotide according to the present invention.

Particularly preferred recombinant DNA fragments according to the present invention comprise a chimeric promoter selected from the group consisting of SEQ ID NO: 1, SEQ ID NO: 2, SEQ ID NO: 3 and a DNA fragment encoding for a protein selected from the group consisting of enzymes, structural proteins, coenzymes, transporters, antibodies, hormones and regulators. It is further particularly preferred that the protein is an enzyme and the enzyme is selected from the group consisting of the classes EC 5.1.3, EC 5.3.1, EC 2.7.1, EC 2.2.1, and EC 1.1.1, preferably selected from the group consisting of EC 5.1.3.3, EC 5.3.1.5, EC 2.7.1.17, EC 2.2.1.2, EC 2.2.1.1, EC 1.1.1.1, EC 5.3.1.4, EC 2.7.1.16 and EC 5.1.3.4. Within a further particularly preferred embodiment, the protein is selected from the group consisting of SEQ ID NOs 22 to 138.

Within a further embodiment, the present invention provides an expression plasmid comprising at least one recombinant DNA fragment according to the present invention.

The present invention further provides a host cell transformed with at least one recombinant DNA fragment comprising the chimeric promoter according to the present invention. The host cell according to the present invention is preferably used for metabolic engineering or for metabolic transformation of xylose containing substrates to preferred metabolites.

The recombinant host cell according to the present invention is preferably selected from bacteria, yeast, or fungal cells. In a particularly preferred embodiment, the host cell is selected from the group consisting of *Escherichia, Klebsiella, Pseudomonas, Lactobacillus, Bacillus, Streptomyces; Saccharomyces, Kluyveromyces, Schizosaccharomyces, Candida, Yarrowia, Komagataella, Pichia, Hansenula, Penicillium, Trichoderma, Hypocrea, Aspergillus, Cantharellu, Agraicus, Boletos, Pleurotus, Trametes, Phanerochaete, Myceliophthora, Chaetomium, Humicola, Chrysosporium, Talaromyces* and *Neurospora.*

It is particularly preferred to select the host cell from the group consisting of *Lactococcus lactis, Lactobacillus brevis, Bacillus subtilis, Bacillus megaterium, Bacillus lentus, Bacillus amyloliquefaciens, Bacillus licheniformis, Pseudomonas fluorescence, Klebsiella planticola, Escherichia coli, Streptomyces lividans, Saccharomyces cerevisiae, Saccharomyces bayanus, Saccharomyces uravum, Saccharomyces pastorianus, Saccharomyces kudriavzevii, Saccharomyces mikatae, Saccharomyces carlsbergensis, Schizosaccharomyces pombe, Kluyveromyces marxianus, Yarrowina lipolytica, Hansenula polymorpha, Pichia angusta, Komagataella pastoris, Pichia pastoris, Aspergillus niger, Aspergillus oryzae, Trichoderma reesei* and *Myceliophthora thermophila.*

The recombinant host cell according to the present invention may comprise one or more plasmids according to the present invention. In addition or alternatively, the recombinant DNA fragment encoding the chimeric promoter is integrated in the genome of the host cell.

**Examples and Figures**

In the following the present invention is described by the examples and figures. The examples and figures are considered for illustrative purpose only and do not limit the scope of the present invention and claims in any respect.

Example 1: Cloning of the plasmid

The plasmid was constructed by recombination cloning in *5. cerevisiae:* A yeast cell was transformed with a vector that has been linearized by restriction enzyme *Not*I and PCR products which have 45 bp homologous overlap to each other and to the vector. The vector consists of a yeast marker (pUG6 87 to 1559 bp), an *E. coli* marker and origin (pUG19 754 to 2534 bp) and a yeast origin (*S. cerevisiae* S288C chromosome IV 44978 to 449831 and *S. cerevisiae* S288C chromosome II 63156 to 63454 bp). These parts are flanked by the restriction sites *Sap*I, *Sbf*I, *Stu*I and *Not*I, respectively. The PCR fragments contained the functional parts (SEQ ID NO.1, SEQ ID NO.2, SEQ ID NO.3, SEQ ID NO.14, SEQ ID NO.16, SEQ ID NO.17, SEQ ID NO.18, SEQ ID NO.19, SEQ ID NO.20 or SEQ ID NO.21, respectively; SEQ ID NO.13 and *S .cerevisiae* S288C chromosome XI 326407 to 326108 bp).

The fragments are assembled by homologous recombination in the yeast cell forming a circular plasmid.

DNA was then isolated from the yeast cell and transformed into *E. coli* to singularize and produce the plasmid in higher amounts. After verification of the plasmid, the yeast strain Simi White™ (Lot: 02905340230601V, Lallemand, Canada) was transformed with the reisolated plasmid with the high-efficiency LiAc method according to Gietz and Schiestl (High-efficiency yeast transformation using the LiAc/SS carrier DNA/PEG method. Not Protocols 2(1), 2007: 31-34).

Example 2: Transcript level - comparison of different plasmids containing different oligonucleotides

The yeast strains harboring the different plasmids were cultivated in 20 ml of glucose-, ethanol- or xylose-containing substrate (10 g/l yeast extract, 20 g/l peptone, 20 g/l carbon source, 200 mg/l G418) in 100 ml shake flask at 30°C and 250 rpm. The cells were harvested by centrifugation at a culture density of approximately OD600 2 and washed with water two times. After that, the cells were resuspended in water and the $OD_{600}$ set to 6. Aliquots of 600 $\mu$L of the cell suspension were centrifuged and the cell pellets stored at -80°C.

The total RNA was extracted from the cell pellets by using the RNeasy Mini Kit™ (Qiagen Germany) according to producer manual. Then 500 ng RNA were used in a reverse transcription reaction to generate cDNA using the iScript Reverse Transcription Supermix for RT-qPCR (BIO RAD Germany) according to producer manual. Transcript levels were determined by using the iQTM SYBR® Green Supermix and the iQ™ iCycler (BIO RAD Germany) following the producer information. ACT1 served as a reference gene for the calculation of XylA mRNA levels. In the qPCR, 225 and 236 bp tall PCR products were amplified from ACT1 and XylA mRNA, respectively. The transcript levels of XylA under the control of different promoters were calculated relative to the transcript level of XylA under control of the pPGK1 promotor of *5. cerevisiae* by the using the $2^{(-\Delta\Delta Ct)}$ method.

Transcript levels were determined for pCHI3, pCHI4 and pCHI5 and are shown in Fig. 4A to 4C. pCHI3 results in an increase of XylA transcription, if *5. cerevisiae* is grown on xylose; when grown on glucose or ethanol, transcription of XylA is detectable in a lower amount. pCHI4 likewise results in an increase of XylA transcription, if *5. cerevisiae* is grown on xylose; when grown on glucose or ethanol, transcription of XylA is detectable in a lower amount. pCHI5 depicts an increase in XylA transcription, if *5. cerevisiae* is grown on glucose; when grown on xylose or ethanol, transcription of XylA is low (Fig. 4A to 4C).

In Fig. 5 the transcript levels of XylA dependent on the chimeric promoters pCHI3, pCHI4 and pCHI5 are compared confirming an increase in the transcript level of XylA via pCHI3 and pCHI4 grown on xylose and an increase in the transcript level of XylA via pCHI5 grown on glucose.

Example 3: Enzyme activity - comparison of different plasmids containing different oligonucleotides

The yeast strains harboring the different plasmids were cultivated in a culture volume of 50 ml in 250 ml shake flasks as defined in example 2 and were harvested at approximately OD600 2. Afterwards the pellet of the culture was stored at -80°C.

The thawed pellets were suspended in 400 $\mu$! buffer (100 mM Tris pH 7.5, 10 mM $MgCl_2$) and homogenized. After the cell lysis the crude extracts were diluted to a total protein concentration of 2 $\mu$g/$\mu$l (measured by Bradford assay). The xylose isomerase activity assays were performed in 100 $\mu$l with 10 % of the diluted crude extracts, 0.25 mM NADH, 3 U/ml sorbitol dehydrogenase and 500 mM Xylose. The reaction kinetics were followed photometrically at 340 nm.

The activity of the xylose isomerase (XI) encoded by XylA and expressed under the control of pCHI3, pCHI4 or pCHI5 was determined for a microorganism such as *5. cerevisiae* grown on glucose, xylose or ethanol and is shown in Fig. 6A to 6C.

A comparison of transcript level vs. enzyme activity is shown in Fig. 7. It shows an increase in the transcript level via pCHI3 and pCHI4 when grown on xylose and via pCHI5 when grown on glucose. Fig. 7 depicts that the correlating enzyme activity for pCHI3 and pCHI4 is strongest when the microorganism is grown on xylose compared to when the microorganism is grown on glucose or ethanol. The correlating enzyme activity for pCHI5 is strongest when the microorganism is grown on glucose compared to when the microorganism is grown on xylose or ethanol. This confirms that chimeric promoters of the present invention allow, by choice of the promoter, to achieve a selectively increased or decreased transcription and correlating enzyme activity and thereby adaption of both to specific conditions such as carbon sources.

## Brief description of the figures

Fig. 1      shows oligonucleotides and parts thereof forming a chimeric promoter of the present invention such as a chimeric promoter of SEQ ID NO.1 (pCHI3), SEQ ID NO.2 (pCHI4) or SEQ ID NO.3 (pCHI5), and oligonucleotides such as promoters and parts thereof regulating genes of glycolysis and gluconeogenesis native to, i.e., originating from *Kluyveromyces lactis.*

Fig. 2A to 2C      show enrichment of transcription factor binding site in chimeric promoters of SEQ ID NO.1, SEQ ID NO.2 or SEQ ID NO.3 of the present invention in comparison to transcription factor binding sites in the respective native (wildtype) promoters.

Fig. 3A to 3C      depict the transcription factor binding sites in more detail, i.e., based on the sequence of the chimeric promoter of SEQ ID NO.1, SEQ ID NO.2 or SEQ ID NO.3 the location and sequences of the transcription binding sites are indicated.

Fig. 4A to 4C      depict transcript levels of XylA in a microorganism such as *5. cerevisiae* depending on the carbon source for growth and the promoter controlling transcription of XylA. The graphs show the XlyA transcript levels for pCHI3, pCHI4 and pCHI5, respectively, in comparison to the XylA transcript levels for the native promoters, of which parts (oligonucleotides) are forming the respective chimeric promoter and the XylA transcript level for a promoter of the state of the art, e.g., the native promoter of PGK1 of *5. cerevisiae.*

Fig. 5      shows a comparison of transcript levels of XylA in cells grown on glucose, xylose or ethanol, where transcription controlled by pCHI3 and pCHI4 depict an increase when cells were grown on xylose and transcription controlled by pCHI5 shows an increase when cells were grown on glucose.

Fig. 6A to 6C      depict activity levels of xylose isomerase (XI) in a microorganism such as *5. cerevisiae* depending on the carbon source for growth and the promotor controlling XylA transcription. The graphs show the XI activity levels for pCHI3, pCHI4 and pCHI5, respectively, in comparison to the XI activity levels for the native promoters, of which parts are forming the respective chimeric promoter and the XI activity levels for a promotor of the state of the art, e.g., the native promotor of PGK1 of *5. cerevisiae.*

Fig. 7      depicts a comparison of the correlation of transcript levels vs. enzyme activity for pCHI3, pCHI4 and pCHI5, respectively, for cells grown on glucose, xylose or ethanol.

## Claims

1. Chimeric promoter **characterized in that** it comprises two or more oligonucleotide sequence(s) or parts thereof regulating the transcription of a gene of an anabolic and/or of a catabolic pathway and increases the transcript level of an RNA typed as messenger RNA fragment encoding for a protein selected from the group consisting of enzymes, structural proteins, coenzymes, transporters, antibodies, hormones and regulators, as regulatory RNA fragment, as enzymatically active RNA fragment or as transfer RNA fragment, said chimeric promoter selected from the group consisting of SEQ ID NO.2, SEQ ID NO.3 or SEQ ID NO.1.

2. Chimeric promoter according to claim 1, wherein the transcript level of the RNA fragment in a yeast host cell is increased when growing the yeast host cell transformed with at least one recombinant DNA fragment comprising the chimeric promoter on a carbon source selected from the group consisting of glucose, xylose, ethanol and combinations thereof.

3. Chimeric promoter according to any of the foregoing claims, wherein the enzyme is a carbohydrate modifying enzyme or a transporter.

4. Chimeric promoter according to any of the foregoing claims, wherein the enzyme is a carbohydrate modifying enzyme selected from the group consisting of EC 5.1.3, EC 5.3.1, EC 2.7.1, EC 2.2.1, EC 1.1.1, EC 5.3.1.4, EC 2.7.1.16, EC 5.1.3.4 and a combination thereof, or a transporter selected from TC 2.A.1.1, TC 2.A.1.2 and a combination thereof.

5. Chimeric promoter according to any of the foregoing claims, wherein the promoter comprises transcription binding factors selected from the group consisting of REB1, GCR1, GCR2, PHD1, TYE7, PHO2, PHO4, AZF1 and a combination thereof.

6. A recombinant DNA fragment comprising the chimeric promoter of any of claims 1 to 5.

7. An expression plasmid comprising at least one recombinant DNA fragment according to claim 6.

8. A host cell transformed with at least one recombinant DNA fragment according to claim 6 or transformed with at least one expression plasmid according to claim 7.

**Patentansprüche**

1. Chimärer Promotor, **dadurch gekennzeichnet, dass** er zwei oder mehr Oligonukleotidsequenzen oder Teile davon umfasst, die die Transkription eines Gens eines anabolen und/oder eines katabolen Weges regulieren und den Transkriptionsspiegel einer RNA erhöhen, die typisiert ist als messenger-RNA-Fragment und für ein Protein codiert, das ausgewählt ist aus der Gruppe bestehend aus Enzymen, Strukturproteinen, Coenzymen, Transportern, Antikörpern, Hormonen und Regulatoren, als regulatorisches RNA-Fragment, als enzymatisch aktives RNA-Fragment oder als Transfer-RNA-Fragment, wobei der chimäre Promotor ausgewählt ist aus der Gruppe bestehend aus SEQ ID NO.2, SEQ ID NO.3 oder SEQ ID N0.1.

2. Chimärer Promotor nach Anspruch 1, wobei der Transkriptionsspiegel des RNA-Fragments in einer Hefewirtszelle erhöht wird, wenn die Hefewirtszelle, die mit mindestens einem rekombinanten DNA-Fragment transformiert ist, das den chimären Promotor umfasst, auf einer Kohlenstoffquelle wächst, die ausgewählt ist aus der Gruppe bestehend aus Glucose, Xylose, Ethanol und Kombinationen davon besteht.

3. Chimärer Promotor nach einem der vorhergehenden Ansprüche, wobei das Enzym ein kohlenhydratmodifizierendes Enzym oder ein Transporter ist.

4. Chimärer Promotor nach einem der vorhergehenden Ansprüche, wobei das Enzym ein kohlenhydratmodifizierendes Enzym ist, das ausgewählt ist aus der Gruppe bestehend aus EC 5.1.3, EC 5.3.1, EC 2.7.1, EC 2.2.1, EC 1.1.1, EC 5.3.1.4, EC 2.7.1.16, EC 5.1.3.4 und einer Kombination davon, oder ein Transporter, ausgewählt aus TC 2.A.1.1, TC 2.A.1.2 und einer Kombination davon.

5. Chimärer Promotor nach einem der vorhergehenden Ansprüche, wobei der Promotor Transkriptionsbindungsfaktoren umfasst, die ausgewählt sind aus der Gruppe bestehend aus REB1, GCR1, GCR2, PHD1, TYE7, PHO2, PHO4, AZF1 und einer Kombination davon.

6. Rekombinantes DNA-Fragment, umfassend den chimären Promotor nach einem der Ansprüche 1 bis 5.

7. Expressionsplasmid, umfassend mindestens ein rekombinantes DNA-Fragment nach Anspruch 6.

8. Wirtszelle, die mit mindestens einem rekombinanten DNA-Fragment nach Anspruch 6 transformiert worden ist oder mit mindestens einem Expressionsplasmid nach Anspruch 7 transformiert worden ist.

**EP 3 690 052 B1**

**Revendications**

1. Promoteur chimérique **caractérisé en ce qu'**il comprend deux séquences d'oligonucléotide ou plus ou des parties correspondantes régulant la transcription d'un gène d'une voie anabolique et/ou d'une voie catabolique et augmente le taux de transcription d'un ARN typé comme un fragment d'ARN messager codant pour une protéine choisie dans le groupe constitué par des enzymes, des protéines structurales, des coenzymes, des transporteurs, des anticorps, des hormones et des régulateurs, comme fragment d'ARN régulateur, comme fragment d'ARN enzymatiquement actif ou comme fragment d'ARN de transfert, ledit promoteur chimérique étant choisi dans le groupe constitué par la SEQ ID NO: 2, la SEQ ID NO: 3 ou la SEQ ID NO: 1.

2. Promoteur chimérique selon la revendication 1, le taux de transcription du fragment d'ARN dans une cellule hôte de levure étant augmenté lors de la croissance de la cellule hôte de levure transformée par au moins un fragment d'ADN recombinant comprenant le promoteur chimérique sur une source de carbone choisie dans le groupe constitué par le glucose, le xylose, l'éthanol et des combinaisons correspondantes.

3. Promoteur chimérique selon l'une quelconque des revendications précédentes, l'enzyme étant une enzyme de modification de glucide ou un transporteur.

4. Promoteur chimérique selon l'une quelconque des revendications précédentes, l'enzyme étant une enzyme de modification de glucide choisie dans le groupe constitué par EC 5.1.3, EC 5.3.1, EC 2.7.1, EC 2.2.1, EC 1.1.1, EC 5.3.1.4, EC 2.7.1.16, EC 5.1.3.4 et une combinaison correspondante, ou un transporteur choisi parmi TC 2.A.1.1, TC 2.A.1.2 et une combinaison correspondante.

5. Promoteur chimérique selon l'une quelconque des revendications précédentes, le promoteur comprenant des facteurs de liaison de transcription choisis dans le groupe constitué par REB1, GCR1, GCR2, PHD1, TYE7, PHO2, PHO4, AZF1 et une combinaison correspondante.

6. Fragment d'ADN recombinant comprenant le promoteur chimérique selon l'une quelconque des revendications 1 à 5.

7. Plasmide d'expression comprenant au moins un fragment d'ADN recombinant selon la revendication 6.

8. Cellule hôte transformée par au moins un fragment d'ADN recombinant selon la revendication 6, ou transformée par au moins un plasmide d'expression selon la revendication 7.

## Fig. 1: Chimeric promoters pCHI3 (SEQ IDNO:1), pCHI4 (SEQ IDNO:2) and pCHI5 (SEQ IDNO:3) and wildtype promoters from *K. lactis*

Fig. 2A to 2C: Transcription factor binding sites in the chimeric promoters of SEQ ID NO:1, SEQ ID NO:2 and SEQ ID NO:3 and native promoters from *K. lactis*

2A) pCHI3

2B) pCHI4

## 2C) pCHI5

EP 3 690 052 B1

19

Fig. 3A to 3C: Sequences of transcription factor binding sites in the sequences of chimeric promoters of SEQ IDNO:1, SEQ IDNO:2 and SEQ ID NO:3

**3A) pCHI3**

```
        1          10          20          30          40          50          60          70          80          90
AAATGATCAC AAATGTGATT GATGATTTGA CACGACTAGA AAAGAGAACG AAAAAGGGAA ATTCCATGTC ACGTGCGTTG GCACGTGACA
                                              pK5_a
                                                                              TYE7-bs              TYE7-bs
                                                                              PHO4-bs              PHO4-bs

       100         110         120         130         140         150         160         170         180
TGGAATATCG AAGAAAGAAA AAAAAAAACG ATCTCGTCCT AGTGGAAGCC CAGAGTCTGG TCCCCCCGGA GTCTTCCCAA AACAAGAAGC
                                              pK5_a
                                   GCR1-bs                               GCR1-bs
                                   GCR2-bs

       190         200         210         220         230         240         250         260         270
TGACACATGT TGACACAGAA CACCCCACAG CAAATGCACC ACGCTACGTA GATCAGGAAG CTTAACTCTA GCGACCTGTC GCTCGCCCCA
                                              pK5_a
                                   GCR1-bs

       280         290         300         310         320         330         340         350         360
CAGAACCTCA CCCGAGAACC ACACATTACA CGCCGCCAGC TCCCACTATA CTCATCTTGC TTCCCTTAAG CGTTCTCACG ATTCGTTCGC
                                              pK5_a
                                        GCR1-bs

       370         380         390         400         410         420         430         440         450
TGGATAATTA TCTTCTTCCG TCTATCTTCT TCTAAATTGA TCAGTATCAT AATATGGAAA AAAAGGTTGT TCGTGAATTT TTCCTCCATT
                                              pK2_b
                        GCR1-bs            PHO2-bs
pK5_a    PHO2-bs   PHO2-bs                 PHO2-bs

       460         470         480         490         500         510         520         530         540
CAATTCATAT ATAATAAGAA CAATGAATTG CATACTTCCC AAATCTTGAT AATTCGTTTT AATATCTATC AGTAGTTGTT TATATTCAAT
                                              pK2_b
                   PHO2-bs      GCR1-bs       PHO2-bs           PHO2-bs          PHO2-bs
        TATA-Box

       550         560         570         580         590         600         610         620         630
TATATCTTGT GTATACAAAA CCATCATCCA AAAACCAATC CAAGTTTCGT TTGACTTATA ACCAATAGTA CCAAAAATTA AACCTTAATT
                                              pK2_b
        PHO2-bs

       640         650         660         670         680         690         701
TTGAAGTACT TTATAATTTA AACTTAATAC GAATTAAACC AAAAAACCCA CAACACGTAA AAGAAATTAC A
                                              pK2_b
        PHO2-bs
```

**3B) pCHI4**

```
1    TTACTTTCCC GGATAATTTC GAAGAGGATA TTACCCGCTT ACGCCAGATG CATGCGAACT TGATGCAGCA CATGAAGAGA CAATCAACAG AGACCCCAGG  100
101  AAATCTTGAA GAACAACAGA AACACATCAA TGATATCGTA GATACCATTG AGAGATATAA CTGAATAAAT ACTTCAAATC ACGTGATGAA TCACGTGCCA  200
201  CAATTACCCT GACTTTTTGT TTACGCAGCA AACATGCAGC ATCCACTAAC TTCAGAGATT CCTGTAAGAT AAGTGGTTCG TTATTTTCCG GATTCCAATT  300
301  TTGGTGGTGC TCCGAAAAGT GGAAGCTCGT GATCCTACAT GCTCACGAAA CCCATCATCA TGCAATCCAC ATTAAAGGAA GGGAAAAGGA TATTGAACTT  400
401  TTGACTATTT AGTATAAATG AAAAACTACTT TGTAAGCTTG GACAGAGGAA TAATTTCTGA TTCGTGCTTC TGCTTCTACT GACTTGCAAA TTTACATATA  500
501  AATATACGTC AAAAGGGGAT TCATTAATTA GAAAAATTCTC TTTTTCAATA GTTGCTATTC ATTATCAATC AATTGGTTAT TATTTTCATC  600
601  TTTTTGTCAT CCTAAACCAT CAACAATATT TAAAATATATC TGTTGCTACA TTAAGAGTTA CTTCAGAAAT AACAAAAAAA TCGATCAAGA ATTAATAAAA  700
```

3C) pCHI5

Fig. 4A to 4C: Transcript levels of XylA dependent on chimeric promoter and carbon source

4A) pCHI3

transcipt level - pCHI3

4B) pCHI4

transcipt level - pCHI4

EP 3 690 052 B1

transcipt level - pCHI5

Fig. 5: Comparison of XylA transcript levels dependent on pCHI3, pCHI4 and pCHI5 and carbon sources selected from glucose, xylose and ethanol

transcript level

Fig. 6A to 6C: Enzyme activity of XI dependent on chimeric promoter and carbon source

6A) pCHI3

EP 3 690 052 B1

enzyme activity - pCHI4

6C) pCHI5

Fig. 7: Comparison correlation of transcript levels and enzyme activity for pCHI3, pCHI4 and pCHI5 dependent on chimeric promoter and carbon sources glucose, xylose and ethanol, respectively

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GIETZ ; SCHIESTL.** High-efficiency yeast transformation using the LiAc/SS carrier DNA/PEG method. *Not Protocols,* 2007, vol. 2 (1), 31-34